# EUROPÄISCHE PATENTSCHRIFT

(11) **EP 3 764 833 B1**
(45) Veröffentlichungstag und Bekanntmachung des Hinweises auf die Patenterteilung: **26.01.2022**
(21) Anmeldenummer: 19704743.4
(22) Anmeldetag: 30.01.2019
(51) Int. Cl.: A44B 11/04, A61F 9/02

(54) **GURTBANDVORRICHTUNG MIT EINSTELLBARER LÄNGE UND FUNKTIONSVORRICHTUNG MIT EINER GURTBANDVORRICHTUNG**
WEBBING STRAP DEVICE OF ADJUSTABLE LENGTH AND FUNCTIONAL DEVICE HAVING A WEBBING STRAP DEVICE
DISPOSITIF DE SANGLE RÉGLABLE EN LONGUEUR ET DISPOSITIF FONCTIONNEL COMPRENANT UN DISPOSITIF DE SANGLE

(30) Priorität: 15.03.2018 DE 102018204007
(43) Veröffentlichungstag der Anmeldung: 20.01.2021
(73) Patentinhaber: Uvex Arbeitsschutz GmbH, 90766 Fürth (DE)
(72) Erfinder: NEUBAUER, Bernd, 90451 Nürnberg (DE); SCHEMM, Daniel, 90579 Langenzenn (DE); FRIEDLEIN, Uwe, 91448 Emskirchen (DE)
(74) Vertreter: Rau, Schneck & Hübner Patentanwälte Rechtsanwälte PartGmbB
(86) Internationale Anmeldenummer: PCT/EP2019/052268
(87) Internationale Veröffentlichungsnummer: WO 2019/174808

(56) Entgegenhaltungen:
- EP-A2- 2 380 452
- DE-A1- 10 007 979
- US-A- 4 815 174

## Beschreibung

Die Erfindung betrifft eine Gurtbandvorrichtung mit einstellbarer Länge. Ferner betrifft die Erfindung eine Funktionsvorrichtung mit einer derartigen Gurtbandvorrichtung.

Gurtbandvorrichtungen sind aus dem Stand der Technik allgemein bekannt. Diese umfassen für gewöhnlich einen Stellband-Anker, einen Stellschlaufen-Anker, einen leiterförmigen Schieber mit einem ersten Rahmensteg, einem zu dem ersten Rahmensteg beabstandeten zweiten Rahmensteg und einem einzigen zwischen dem ersten Rahmensteg und dem zweiten Rahmensteg angeordneten Zwischensteg sowie ein an dem Zwischensteg und an dem Stellband-Anker befestigtes Gurtband. Das Gurtband erstreckt sich zum Ausbilden einer Stellschlaufe zwischen dem ersten Rahmensteg und dem Zwischensteg durch den Schieber hindurch. Zwischen dem ersten Rahmensteg und dem Zwischensteg durchdringt das Gurtband den Schieber dabei doppelt. Für eine automatisierte Herstellung der Gurtbandvorrichtung ist eine mehrfache Durchführung des Gurtbands durch den Schieber hinderlich. Der Durchfädelvorgang ist komplex und im Betrieb wenig robust. Eine solche Gurtbandvorrichtung ist beispielsweise bekannt aus der US D477, 347 S.

Gurtbandvorrichtungen sind ferner bekannt aus der US 4,815,174 A. Derartige Gurtbandvorrichtungen umfassen einen leiterförmigen Schieber mit einem ersten Rahmensteg, an dem ein Gurtband mittels eines Keilstücks reversibel befestigbar ist. Derartige Keilverbindungen neigen bei wechselnden Belastungen des Gurtbands, insbesondere bei zwischenzeitlicher Lastfreiheit, zum Lösen. Die automatisierte Herstellung einer derartigen Klemmverbindung ist zudem technisch aufwendig.

Alternative Ausführungsformen von Gurtbandvorrichtungen sind beispielweise bekannt aus der DE 10 2012 014 693 A1, der US 2,022,483 A, der EP 1 389 434 A1, der DE 100 07 979 A1 und der EP 2 380 452 A2.

Der Erfindung liegt die Aufgabe zugrunde, eine verbesserte Gurtbandvorrichtung bereitzustellen. Die Gurtbandvorrichtung soll in einem robusten Prozess besonders wirtschaftlich und effizient herstellbar sein.

Diese Aufgabe wird durch eine Gurtbandvorrichtung mit den Merkmalen des Anspruchs 1 gelöst. Der Kern der Erfindung liegt darin, dass der Schieber mindestens zwei zwischen dem ersten Rahmensteg und dem zweiten Rahmensteg angeordnete Zwischenstege aufweist und dass das Gurtband zum Ausbilden der Stellschlaufe zwischen den mindestens zwei Zwischenstegen durch den Schieber hindurchgeführt ist, wobei der Schieber mindestens drei Bandöffnungen aufweist, welche von dem Gurtband höchstens einmal durchdrungen sind und wobei das Gurtband zum Befestigen an dem ersten Rahmensteg eine Schieberschlaufe aufweist. Das Gurtband ist dabei an dem ersten Rahmensteg und an dem Stellband-Anker befestigt. Eine mehrfache Durchdringung des Gurtbandes durch den Schieber zwischen dem ersten Rahmensteg und den mindestens zwei Zwischenstegen kann somit vermieden werden. Vorteilhalft wird hierdurch erreicht, dass die erfindungsgemäße Gurtbandvorrichtung automatisierbar und damit besonders wirtschaftlich herstellbar ist.

Die Schieberschlaufe kann an einem Ende des Gurtbandes, insbesondere durch eine Vernähung und/oder eine Verschweisung und/oder eine Verklebung eines Schnittendes des Gurtbandes, mit einem von dem Schnittende beabstandeten Teils des Gurtbandes, ausgebildet sein. Zum Befestigen der Schieberschlaufe an dem ersten Rahmensteg, kann das Gurtband im Bereich der Schieberschlaufe durch die erste Bandöffnung hindurchgeführt und dann zu einer Fixierstelle, insbesondere zu einer Naht- und/oder Schweiß- und/oder Klebestelle, zurückgeführt sein. Hierdurch kann die Verbindung des Gurtbandes mit dem Stellband-Anker formschlüssig und damit besonders sicher erfolgen.

Vorzugsweise sind die beiden Enden des ersten Rahmenstegs und/oder des zweiten Rahmenstegs mit je einem Rahmenholm verbunden. Die beiden Rahmenholme können zusammen mit dem ersten Rahmensteg und dem zweiten Rahmensteg einen geschlossenen Schieberrahmen ausbilden. Vorzugsweise sind auch die mindestens zwei Zwischenstege an ihren beiden Enden jeweils mit einem der beiden Rahmenholme verbunden. Die mindestens zwei Zwischenstege können insbesondere auch nur einseitig mit einem der Rahmenholme verbunden sein. Insbesondere kann zumindest ein Teil der Rahmenstege, der mindestens zwei Zwischenstege und der Rahmenholme eine Unterbrechung zur Durchführung des Gurtbandes aufweisen. Vorzugsweise sind der erste Rahmensteg und/oder der zweite Rahmensteg und/oder die mindestens zwei Zwischenstege parallel zueinander orientiert. Die Führung des Gurtbands durch den Schieber ist somit besonders gleichmäßig und ein Verkippen des Schiebers kann zuverlässig verhindert werden.

Angrenzend an den ersten Rahmensteg und zwischen diesem und einem ersten der mindestens zwei Zwischenstege weist der Schieber eine erste Bandöffnung zur Durchführung des Gurtbands auf. Eine zweite Bandöffnung zur Durchführung des Gurtbands weist der Schieber angrenzend an den zweiten Rahmensteg und zwischen diesem und einem zweiten der mindestens zwei Zwischenstege auf. Zwischen je zwei benachbart zueinander angeordneten Zwischenstegen weist der Schieber mindestens eine weitere, nämlich mittlere, Bandöffnung auf. Der Schieber kann mindestens drei, insbesondere mindestens vier, insbesondere mindestens fünf, Zwischenstege aufweisen.

Vorzugsweise weist der Schieber genau zwei Zwischenstege auf. Der Schieber weist somit vorzugsweise insgesamt genau drei Bandöffnungen auf. Besonders bevorzugt ist das Gurtband durch die erste Bandöffnung und durch die zweite Bandöffnung und durch die mittlere Bandöffnung hindurchgeführt.

Das Gurtband kann an dem ersten Rahmensteg und/oder an dem Stellband-Anker kraftschlüssig, insbesondere in Form einer Klemmverbindung und/oder einer Schraubverbindung, und/oder formschlüssig, insbesondere in Form einer Kederverbindung, und/oder einer Stiftverbindung und/oder einer Vernähung und/oder stoffschlüssig, insbesondere durch Verkleben und/oder Verschweißen, angebracht sein. Das Gurtband ist somit besonders fest und zuverlässig mit dem ersten Rahmensteg und/oder dem Stellband-Anker verbunden.

Das Gurtband kann aber insbesondere auch mehrteilig ausgebildet sein. Vorzugsweise ist das Gurtband zweiteilig ausgebildet, wobei zwischen einem ersten Teil und einem zweiten Teil des Gurtbandes ein Verschlussmittel angeordnet ist. Das Verschlussmittel kann zum reversiblen Verbinden des ersten Teils und des zweiten Teils des Gurtbands eine Rastverbindung aufweisen. Vorteilhaft wird hierdurch erreicht, dass die Gurtbandvorrichtung in einer Offenstellung und einer Verschlussstellung anordenbar ist.

Das Gurtband kann ein Gewebe und/oder ein Gestricke und/oder ein Gewirke aufweisen. Vorzugsweise besteht das Gurtband vollständig aus einem gewebten und/oder gestrickten und/oder gewirkten textilen Material.

Vorzugsweise ist das Verschlussmittel derart ausgebildet, dass es durch mindestens eine, insbesondere sämtliche, der Bandöffnungen hindurchführbar ist. Das Gurtband ist somit besonders einfach austauschbar.

Vorzugsweise durchläuft das Gurtband sämtliche der Bandöffnungen, insbesondere die erste Bandöffnung und die zweite Bandöffnung und die mindestens eine mittlere Bandöffnung. Die Reibung zwischen dem Gurtband und dem Schieber ist somit erhöht und ein unerwünschtes Verlagern des Gurtbandes relativ zu dem Schieber wird zuverlässig verhindert.

Vorzugsweise weist das Gurtband kein loses Ende auf. Unter einem losen Ende wird ein mit der Länge der Gurtbandvorrichtung in seiner Erstreckung variierendes Ende verstanden. Vorzugsweise sind beide Enden des Gurtbandes an dem Stellband-Anker und/oder an dem Stellschlaufen-Anker und/oder an dem Schieber angebracht und somit keine losen Enden. Durch die Vermeidung derartiger loser Enden wird der Tragekomfort der Gurtbandvorrichtung zusätzlich erhöht.

Eine Gurtbandvorrichtung gemäß Anspruch 2 ist besonders zuverlässig einstellbar und verstellsicher. Dadurch, dass sich das Gurtband sowohl durch die mindestens eine mittlere Bandöffnung als auch durch die zweite Bandöffnung hindurch erstreckt, ist die Reibung zwischen dem Gurtband und dem Schieber erhöht. Ein unbeabsichtigtes Verlagern des Gurtbandes relativ zu dem Schieber kann somit verhindert werden. Vorzugsweise entspricht eine Länge der jeweiligen Bandöffnung in etwa einer Breite des Gurtbandes. Eine Breite der ersten Bandöffnung und/oder der zweiten Bandöffnung und/oder der mindestens einen mittleren Bandöffnung beträgt vorzugsweise höchstens 6 mm, insbesondere höchstens 4 mm, insbesondere höchstens 3 mm, insbesondere höchstens 2 mm. Eine Tiefe der ersten Bandöffnung und/oder der zweiten Bandöffnung und/oder der mindestens einen mittleren Bandöffnung beträgt vorzugsweise mindestens 1 mm, insbesondere mindestens 2 mm, insbesondere mindestens 4 mm, insbesondere mindestens 6 mm. Hierdurch kann ein unbeabsichtigtes Verlagern des Gurtbandes relativ zu dem Schieber sicher verhindert werden.

Eine Gurtbandvorrichtung gemäß Anspruch 3 ist automatisiert und wirtschaftlich herstellbar. Unter der Durchdringung wird eine Erstreckung des Gurtbandes durch den Schieber, insbesondere durch eine der Bandöffnungen, verstanden. Vorzugsweise sind zueinander benachbarte Durchdringungen jeweils durch mindestens einen der mindestens zwei Zwischenstege voneinander getrennt. Dadurch, dass das Gurtband jede der Bandöffnungen höchstens und vorzugsweise genau einmal durchdringt, kann die Gurtbandvorrichtung in einem automatisierten Prozess zuverlässig und robust hergestellt werden.

Eine Gurtbandvorrichtung nach Anspruch 4 ist automatisiert und wirtschaftlich herstellbar. Dadurch, dass die mindestens zwei Zwischenstege von dem Gurtband lediglich einseitig umlaufen werden, kann das Gurtband besonders einfach durch den Schieber, insbesondere die Bandöffnungen, hindurchgeführt werden.

Eine Gurtbandvorrichtung nach Anspruch 5 ist besonders wirtschaftlich herstellbar und weist einen hohen Tragekomfort auf. Unter der einstückigen Ausbildung wird verstanden, dass das Gurtband aus einen einzelnen, insbesondere fügenahtlosem, Band besteht. Das einstückige Gurtband weist insbesondere keine Klebenaht und/oder keine Vernähung, auf, über die es aus mehreren Teilstücken zusammengesetzt ist. Durch die Vermeidung derartiger Fügenähte weist die Gurtbandvorrichtung einen besonders hohen Tragekomfort auf.

Eine Gurtbandvorrichtung nach Anspruch 6 gewährleistet einen festen Sitz und weist einen hohen Tragekomfort auf. Die Streckdehnung des Gurtbandes beträgt vorzugsweise mindestens 10 %, insbesondere mindestens 20 %, insbesondere mindestens 40 %, insbesondere mindestens 60 %, insbesondere mindestens 100 %. Das Gurtband kann hierzu ein elastisches, insbesondere gummielastisches, Material aufweisen. Das Gurtband kann auch ein elastisches Gewebe und/oder Gestricke und/oder Gewirke aufweisen.

Eine Gurtbandvorrichtung nach Anspruch 7 ist im Gebrauch besonders robust. Die Ankerschlaufe kann an einem Ende des Gurtbandes, insbesondere durch eine Vernähung und/oder eine Verschweißung und/oder eine Verklebung eines Schnittendes des Gurtbandes, mit einem von dem Schnittende beabstandeten Teil des Gurtbandes, ausgebildet sein.

Zum Befestigen des Gurtbands an dem Stellband-Anker kann das Gurtband im Bereich der Ankerschlaufe durch eine Stellband-Anker-Öse hindurchgeführt und dann zu einer Fixierstelle, insbesondere zu einer Naht- und/oder Schweiß- und/oder Klebestelle, zurückgeführt sein.

Das Gurtband kann an dem Stellband-Anker auch mittels einer Ankerwulst befestigt sein. Vorzugsweise ist die Ankerwulst durch mehrere, insbesondere übereinander geschichtete und/oder spiralförmig gewickelte, Lagen des Gurtbandes ausgebildet. Die mehreren Lagen des Gurtbandes können miteinander vernäht und/oder stoffschlüssig, insbesondere durch Verkleben oder Verschweißen, miteinander verbunden sein. Zur Verbindung mit dem Stellband-Anker weist die Ankerwulst vorzugsweise eine Wulstdicke auf, welche größer ist als eine Ösenbreite einer Stellband-Anker-Öse des Stellband-Ankers. Die Ösenbreite erstreckt sich senkrecht zu einer Längserstreckung der Stellband-Anker-Öse. Hierdurch kann die Verbindung des Gurtbandes mit dem Stellband-Anker formschlüssig und damit besonders sicher erfolgen.

Alternativ kann die Ankerwulst auch durch einen mit dem Gurtband verbundenen Aufdickungsskörper ausgebildet sein. Die Wulstdicke des Aufdickungskörpers kann größer sein als die Ösenbreite. Der Aufdickungskörper kann die Stellband-Anker-Öse auch entlang ihrer Längserstreckung überragen. Der Aufdickungskörper kann mit dem Gurtband verklemmt und/oder verschweißt und/oder verklebt und/oder formschlüssig verbunden sein.

Entsprechend der Verbindung des Gurtbandes mit dem Stellband-Anker kann auch die Verbindung des Gurtbandes an dem ersten Rahmensteg auf oben genannte Weise mittels einer Ankerwulst erfolgen.

Eine Gurtbandvorrichtung nach Anspruch 8 ist besonders wirtschaftlich herstellbar und im Gebrauch robust. Das Gurtband kann ein textiles Material mit dem thermoplastischen Kunststoff aufweisen. Vorzugsweise ist der thermoplastische Kunststoff an mindestens einem Schnittende des Gurtbands zu einem Schmelzsaum verschmolzen. Hierdurch kann ein Aufdröseln des textilen Gurtbandes verhindert werden. Vorzugsweise ist die Schieberschlaufe und/oder die Ankerschlaufe durch Verschweißen jeweils eines Schnittendes des Gurtbandes mit einem zu diesem Schnittende beabstandeten Teil des Gurtbandes ausgebildet.

Eine Gurtbandvorrichtung nach Anspruch 9 ist wirtschaftlich herstellbar und weist einen hohen Tragekomfort auf. Die Gurtbandvorrichtung kann aus einem plattenförmigen Halbzeug, insbesondere durch trennende und/oder spanende Bearbeitung, hergestellt sein. Durch die Anordnung des ersten Rahmenstegs, des zweiten Rahmenstegs und der mindestens zwei Zwischenstege in der Stegebene bietet die Gurtbandvorrichtung im Bereich des Schiebers eine gleichmäßige Auflagefläche. Hervorstehende Stege könne verhindert werden, woraus ein hoher Tragekomfort resultiert.

Eine Gurtbandvorrichtung nach Anspruch 10 ist automatisiert fertigbar. Die Positionierhilfe ist vorzugsweise als Erhebung, insbesondere als stiftförmige und/oder als stegförmige und/oder als flächige Erhebung, ausgebildet. Die Positionierhilfe kann aber auch als Vertiefung ausgebildet sein. Die Positionierhilfe gewährleistet ein Ausrichten und/oder Festhalten des Schiebers im Herstellungsprozess. Vorzugsweise ist die Positionierhilfe zumindest teilweise, insbesondere vollständig, von dem Gurtband überdeckt.

Die Positionierhilfe kann an mindestens einem der Rahmenholme und/oder an dem mindestens einen Zwischensteg und/oder an dem zweiten Rahmensteg angeordnet sein.

Eine Gurtbandvorrichtung nach Anspruch 11 ist automatisiert fertigbar. Insbesondere ist die Positionierhilfe ausschließlich an dem ersten Rahmensteg angeordnet. Die Positionierhilfe kann einen Anschlag zum Ausrichten und Festhalten des Schiebers bilden.

Eine Gurtbandvorrichtung nach Anspruch 12 gewährleistet eine erhöhte Reibung zwischen dem Gurtband und dem Schieber. Ein unbeabsichtigtes Verlagern des Gurtbandes relativ zu dem Schieber kann somit verhindert werden. Die Steg-Rändelung kann als Längsrändelung und/oder als Kreuzrändelung ausgebildet sein.

Eine Gurtbandvorrichtung nach Anspruch 13 gewährleistet ein einfaches Verlagern des Schiebers relativ zu dem Gurtband. Vorzugsweise ist die Rahmen-Rändelung an einer Außenseite des mindestens einen Rahmenholms angeordnet. Durch die Rahmen-Rändelung kann der Schieber besonders einfach manuell, insbesondere von Hand, relativ zu dem Gurtband verlagert werden.

Der Erfindung liegt ferner die Aufgabe zugrunde, eine Funktionsvorrichtung zu schaffen, welche automatisiert und wirtschaftlich herstellbar ist.

Diese Aufgabe wird durch eine Funktionsvorrichtung mit den Merkmalen des Anspruchs 14 gelöst. Die Vorteile der erfindungsgemäßen Funktionsvorrichtung entsprechen den Vorteilen der bereits beschriebenen Gurtbandvorrichtung. Das Funktionselement kann optional mit dem Stellband-Anker und/oder dem Stellschlaufen-Anker reversibel, das heißt insbesondere lösbar, verbunden sein. Hierdurch wird ein Austausch der Gurtbandvorrichtung ermöglicht. Vorzugsweise sind der Stellband-Anker und/oder der Stellschlaufen-Anker über ein Rastelement mit dem Funktionselement verbunden. Die Verbindung zwischen der Gurtbandvorrichtung und dem Funktionselement ist somit besonders einfach, insbesondere werkzeuglos, öffenbar.

Vorzugsweise ist die Positionierhilfe an einer dem Funktionselement abgewandten Seite des Schiebers angeordnet. Vorzugsweise ist mindestens ein Ende des Gurtbandes, insbesondere im Bereich der Schieberschlaufe und/oder im Bereich der Ankerschlaufe, an einer dem Funktionselement abgewandten Seite der Gurtbandvorrichtung angeordnet. Hierdurch wird jeweils ein besonders hoher Tragekomfort der Funktionsvorrichtung gewährleistet.

Das Funktionselement kann Teil einer Schutzausrüstung, insbesondere eines Körperschutzes, insbesondere einer Schutzkleidung sein. Das Funktionselement kann auch ein Tragelement, insbesondere zum Tragen von Werkzeug und/oder anderen Gebrauchsgegenständen, insbesondere am Körper, sein.

Eine Funktionsvorrichtung nach Anspruch 15 ist besonders wirtschaftlich herstellbar und weist einen hohen Tragekomfort auf. Vorzugsweise ist das Funktionselement dazu ausgebildet, am Kopf getragen zu werden. Ein hoher Tragekomfort der Gurtbandvorrichtung ist hierbei besonders Vorteilhaft. Das Funktionselement kann als Atemschutzmaske und/oder als Schutzbrille, insbesondere als Arbeitsschutzbrille oder als Skibrille, ausgebildet sein.

Weitere Merkmale, Vorteile und Einzelheiten der Erfindung ergeben sich aus der nachfolgenden Beschreibung eines Ausführungsbeispiels. Es zeigen:
- Fig. 1: eine perspektivische Darstellung einer erfindungsgemäßen Funktionsvorrichtung mit einem Funktionselement und einer daran angebrachten Gurtbandvorrichtung,

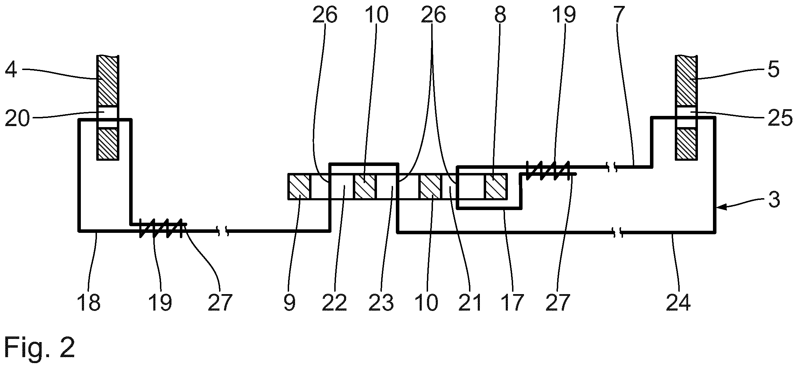

- Fig. 2: eine schematische Schnittansicht der Gurtbandvorrichtung gemäß Fig. 1 mit einem Stellband-Anker, einem Stellschlaufen-Anker, einem leiterförmigen Schieber und einem Gurtband,

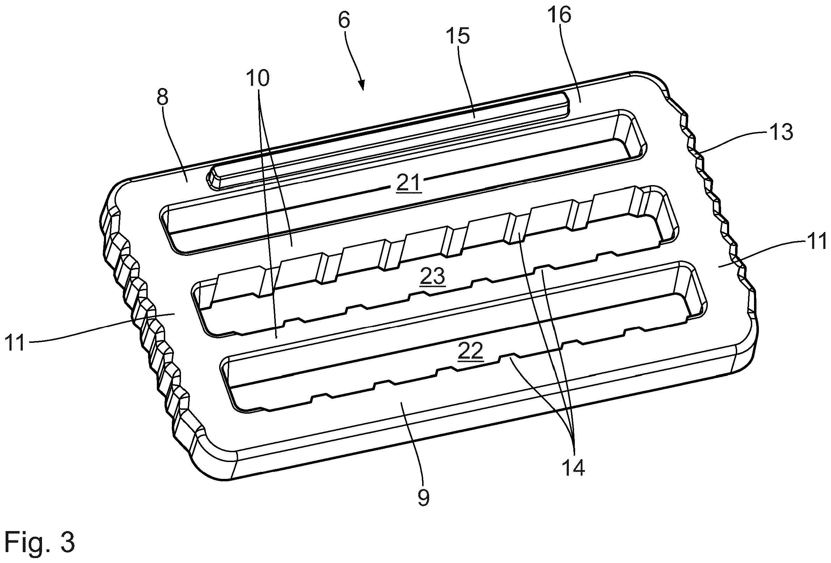

- Fig. 3: eine perspektivische Darstellung des Schiebers der Gurtbandvorrichtung gemäß Fig. 1, und
- Fig. 4: eine schematische Schnittansicht einer Gurtbandvorrichtung mit einem Stellband-Anker, einem Stellschlaufen-Anker, einem leiterförmigen Schieber und einem Gurtband gemäß einem weiteren Ausführungsbeispiel, wonach das Gurtband zum Befestigen an dem Stellband-Anker eine Ankerwulst aufweist.

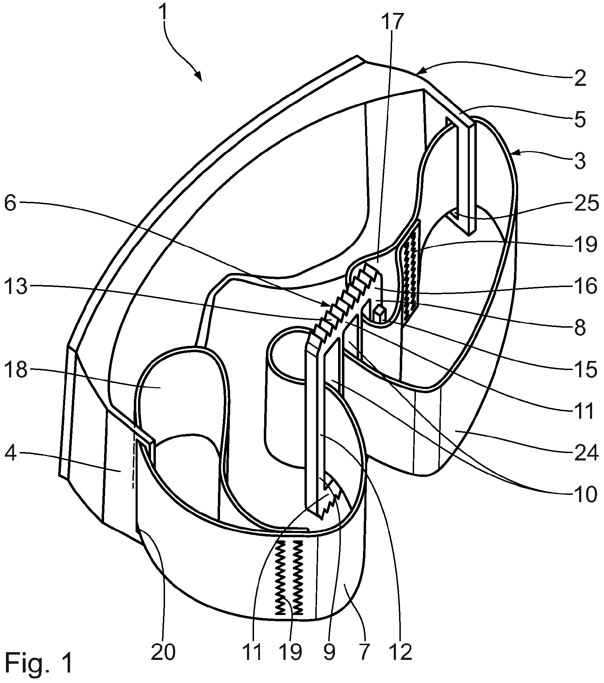

Eine Funktionsvorrichtung 1 gemäß Fig. 1 umfasst ein Funktionselement 2 sowie eine daran angebrachte Gurtbandvorrichtung 3. Das Funktionselement 2 ist als Augenschutzelement ausgebildet. Die Funktionsvorrichtung 1 ist eine Schutzbrille, insbesondere eine Vollsichtbrille.

Die Gurtbandvorrichtung 3 umfasst einen Stellband-Anker 4 und einen Stellschlaufen-Anker 5. Die Gurtbandvorrichtung 3 ist über den Stellband-Anker 4 und den Stellschlaufen-Anker 5 an dem Funktionselement 2 angebracht. Der Stellband-Anker 4 und der Stellschlaufen-Anker 5 sind stoffschlüssig und damit fest mit dem Funktionselement 2 verbunden. Die Gurtbandvorrichtung 3 bildet somit ein Kopfband zur Befestigung der Funktionsvorrichtung 1 am Kopf aus.

Die Gurtbandvorrichtung 3 umfasst des Weiteren einen leiterförmigen Schieber 6 und ein Gurtband 7. Das Gurtband 7 ist mehrfach durch den Schieber 6 hindurchgeführt, wobei der Schieber 6 zum Einstellen einer Länge der Gurtbandvorrichtung 3 zwischen dem Stellband-Anker 4 und dem Stellschlaufen-Anker 5 relativ zu dem Gurtband 7 verlagerbar ist.

Der Schieber 6 umfasst einen ersten Rahmensteg 8, einen zu dem ersten Rahmensteg 8 beabstandeten zweiten Rahmensteg 9 sowie zwei zwischen dem ersten Rahmensteg 8 und dem zweiten Rahmensteg 9 angeordnete Zwischenstege 10. Der erste Rahmensteg 8, der zweite Rahmensteg 9 und die beiden Zwischenstege 10 sind an ihren beiden Enden jeweils mit einem Rahmenholm 11 verbunden. Der erste Rahmensteg 8, der zweite Rahmensteg 9, die beiden Zwischenstege 10 sowie die beiden Rahmenholme 11 sind in einer gemeinsamen Stegebene 12 angeordnet. Die beiden Rahmenholme 11 bilden zusammen mit dem ersten Rahmensteg 8 und dem zweiten Rahmensteg 9 einen vollständig geschlossenen Rahmen aus. Der erste Rahmensteg 8, der zweite Rahmensteg 9 und die beiden Zwischenstege 10 sind parallel zueinander orientiert. Der Schieber 6 ist aus einem, insbesondere thermoplastischen, Kunststoff hergestellt.

Die beiden Rahmenholme weisen jeweils an einer Außenseite des Schiebers 6 eine Rahmen-Rändelung 13 auf. Die jeweilige Rahmen-Rändelung 13 ist derart ausgebildet, dass der Schieber 6 einfach von Hand relativ zu dem Gurtband 7 verlagerbar ist.

Der zweite Rahmensteg 9 sowie die beiden Zwischenstege 10 weisen je eine Steg-Rändelung 14 auf. Die Steg-Rändelung 14 ist jeweils an einer Innenseite des zweiten Rahmenstegs 9 und der beiden Zwischenstege 10, insbesondere in Richtung eines geometrischen Schwerpunkts des Schiebers 6 orientiert, angeordnet. Die Steg-Rändelungen 14 sind derart ausgebildet, dass ein zwischen dem Schieber 6 und dem Gurtband 7 ausgebildeter Reibwert erhöht ist.

An dem ersten Rahmensteg 8 ist eine Positionierhilfe 15 angebracht. Die Positionierhilfe 15 ist in Form einer stegförmigen Erhebung ausgebildet und parallel zu dem ersten Rahmensteg 8 orientiert. Die Positionierhilfe 15 ist an einer dem Funktionselement 2 abgewandten Oberseite 16 des Schiebers 6 angeordnet.

Das Gurtband 7 weist an seinem ersten Ende eine Schieberschlaufe 17 und an seinem zweiten Ende eine Ankerschlaufe 18 auf. Die Schieberschlaufe 17 sowie die Ankerschlaufe 18 sind jeweils durch eine Vernähung 19 ausgebildet. Das Gurtband 7 ist über die Ankerschlaufe 18 an dem Stellband-Anker 4 angebracht. Hierzu ist das Gurtband 7 im Bereich der Ankerschlaufe 18 durch eine Stellband-Anker-Öse 20 des Stellband-Ankers 4 hindurchgeführt und zu einer ersten Nahtstelle zurückgeführt.

Der Schieber 6 weist eine von dem ersten Rahmensteg 8 dem hierzu benachbarten Zwischensteg 10 und den beiden Rahmenholmen 11 umschlossene erste Bandöffnung 21 auf. Umrahmt von dem zweiten Rahmensteg 9, dem hierzu benachbarten Zwischensteg 10 und den beiden Rahmenholmen 11 weist der Schieber 6 eine zweite Bandöffnung 22 auf. Umschlossen von den beiden Zwischenstegen 10 und den beiden Rahmenholmen 11 umfasst der Schieber 6 eine mittlere Bandöffnung 23. Das Gurtband 7 ist über die Schieberschlaufe 17 an dem ersten Rahmensteg 8 angebracht. Hierzu erstreckt sich das Gurtband 7 im Bereich der Schieberschlaufe 17 durch die erste Bandöffnung 21 hindurch und ist zu einer zweiten Nahtstelle zurückgeführt.

Das Gurtband 7 ist durch die mittlere Bandöffnung 23 hindurchgeführt. Zwischen der ersten Bandöffnung 21 und der mittleren Bandöffnung 23 erstreckt sich das Gurtband 7 in Form einer Stellschlaufe 24. Zur verlagerbaren Befestigung des Gurtbands 7 weist der Stellschlaufen-Anker 5 eine Stellschlaufen-Öse 25 auf. Das Gurtband 7 ist im Bereich der Stellschlaufe 24 durch die Stellschlaufen-Öse 25 hindurchgeführt.

Zwischen der mittleren Bandöffnung 23 und der Ankerschlaufe 18 ist das Gurtband 7 durch die zweite Bandöffnung 22 hindurchgeführt. Das Gurtband 7 durchdringt die erste Bandöffnung 21, die zweite Bandöffnung 22 und die mittlere Bandöffnung 23 jeweils genau einmal. Zueinander benachbarte Durchdringungen 26 des Gurtbandes 7 durch den Schieber 6 sind dabei durch jeweils einen der zwei Zwischenstege 10 voneinander getrennt.

Die beiden Zwischenstege 10 werden von dem Gurtband 7 jeweils einseitig umlaufen.

Das Gurtband 7 ist elastisch ausgebildet. Eine Streckdehnung des Gurtbands 7 beträgt mindestens 10 %. Zudem ist das Gurtband 7 einstückig ausgebildet. Das Gurtband 7 ist aus einem textilen Material und vorzugsweise in einem Webprozess hergestellt. Das Gurtband 7 weist einen thermoplastischen Kunststoff auf. Ein Aufdröseln des Gurtbandes 7 ist durch eine Aufschmelzung des thermoplastischen Kunststoffs im Bereich der beiden Schnittenden 27 verhindert.

Die Funktionsweise der Gurtbandvorrichtung 3 mit einstellbarer Länge bzw. der Funktionsvorrichtung 1 mit dieser Gurtbandvorrichtung 3 ist wie folgt:
Zum Befestigen der Funktionsvorrichtung 1 an dem Kopf wird das Funktionselement 2, insbesondere das Augenschutzelement, vor dem Gesicht angeordnet. Die Gurtbandvorrichtung 3 wird um den Kopf herumgelegt, wobei sich diese von dem Stellschlaufen-Anker 5 hinter dem Kopf vorbei zu dem Stellband-Anker 4 erstreckt. Zum Anpassen der Gurtbandvorrichtung 3 an einem jeweiligen Umfang des Kopfes ist die Länge der Gurtbandvorrichtung 3 einstellbar.

Im Bereich der Stellschlaufe 24, zwischen der Schieberschlaufe 17 und dem Stellschlaufenanker 5 ist das Gurtband 7 doppelt geführt. Zwischen dem Stellbandanker 4 und der zweiten Bandöffnung 22 erstreckt sich das Gurtband 7 hingegen nur einfach. Durch Verlagern des Schiebers 6 relativ zu dem Gurtband 7 kann somit die Länge der Gurtbandvorrichtung 3 eingestellt werden. Ein Vergrößern der Stellschlaufe 24 führt dabei zu einer Verringerung der Länge der Gurtbandvorrichtung 3. Ein Verringern der Größe der Stellschlaufe 24 führt zu einer Vergrößerung der Länge der Gurtbandvorrichtung 3.

Die Verlagerung des Schiebers 6 relativ zu dem Gurtband 7 erfolgt manuell, wobei die Rahmen-Rändelungen 13 das Verschieben erleichtern.

Die Länge der Gurtbandvorrichtung 3 wird derart eingestellt, dass das Gurtband 7, insbesondere unter Dehnung desselben, fest an dem Kopf anliegt. Die Steg-Rändelungen 14 gewährleisten eine erhöhte Reibung zwischen dem Schieber 6 und dem Gurtband 7 und verhindern ein ungewolltes Verlagern des Schiebers 6 relativ zu dem Gurtband 7.

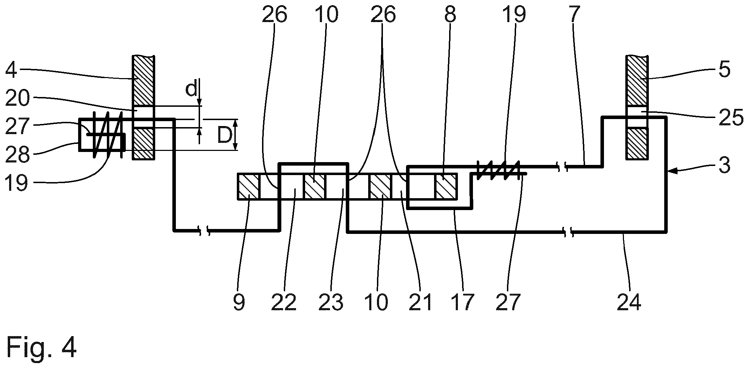

In Fig. 4 ist eine Gurtbandvorrichtung 3 gemäß einem alternativen Ausführungsbeispiel dargestellt. Die Gurtbandvorrichtung 3 unterscheidet sich von der vorstehend beschriebenen Ausführungsform dahingehend, dass das Gurtband 7 an dem Stellband-Anker 4 mittels einer Ankerwulst 28 angebracht ist. Die Ankerwulst 28 umfasst mehrere übereinander geschichtete Lagen des Gurtbandes 7. Die mehreren, übereinander geschichteten Lagen des Gurtbandes 7 sind durch eine Vernähung 19 fest miteinander verbunden. Zur Durchführung des Gurtbandes 7 ist eine Ösenbreite d der Stellband-Anker-Öse 20 größer als eine Dicke des Gurtbandes 7. Eine Wulstdicke D der Ankerwulst 28 ist wiederum größer als die Ösenbreite d. Hierdurch wird gewährleistet, dass das durch die Stellband-Anker-Öse 20 geführte Gurtband 7 formschlüssig mit dem Stellband-Anker 4 verbunden ist. Die Funktionsweise der Gurtband-Vorrichtung 3 gemäß dem alternativen Ausführungsbeispiel entspricht der Funktionsweise der zuvor beschriebenen Gurtband-Vorrichtung 3.

Die erfindungsgemäße Ausbildung der Gurtbandvorrichtung 3 gewährleistet eine Reduktion des Herstellungsaufwands und ermöglicht eine automatisierte Fertigung. Insbesondere dadurch, dass das Gurtband 7 jede der Öffnungen des Schiebers 6, insbesondere die erste Bandöffnung 21, die zweite Bandöffnung 22 und die mittlere Bandöffnung 23, nur einmal durchdringt, kann das einstückige Gurtband 7 automatisiert zwischen dem Stellband-Anker 4, dem Stellschlaufen-Anker 5 und durch den Schieber 6 hindurchgeführt werden. Der Herstellungsprozess der Gurtbandvorrichtung 3 ist dadurch besonders robust und wirtschaftlich.

## Patentansprüche

1. Gurtbandvorrichtung mit einstellbarer Länge, aufweisend
- einen Stellband-Anker (4),
- einen Stellschlaufen-Anker (5) mit
-- einer Stellschlaufen-Öse (25),
- einen leiterförmigen Schieber (6) mit
-- einem ersten Rahmensteg (8),
-- einem zu dem ersten Rahmensteg (8) beabstandeten zweiten Rahmensteg (9),
-- mindestens zwei zwischen dem ersten Rahmensteg (8) und dem zweiten Rahmensteg (9) angeordneten Zwischenstegen (10), und
-- mindestens drei Bandöffnungen (21, 22, 23) zum Durchführen eines Gurtbands (7), und
- ein an dem ersten Rahmensteg (8) und an dem Stellband-Anker (4) befestigtes Gurtband (7),
- wobei sich das Gurtband (7) zum Ausbilden einer Stellschlaufe (24) zwischen den mindestens zwei Zwischenstegen (10) durch den Schieber (6) hindurch erstreckt, und
- wobei sich das Gurtband (7) im Bereich der Stellschlaufe (24) durch die Stellschlaufen-Öse (25) hindurch erstreckt,
**dadurch gekennzeichnet, dass**
das Gurtband (7) jede der Bandöffnungen (21, 22, 23) höchstens einmal durchdringt und zum Befestigen an dem ersten Rahmensteg (8) eine Schieberschlaufe (17) aufweist.

2. Gurtbandvorrichtung nach Anspruch 1, **dadurch gekennzeichnet, dass** sich das Gurtband (7) angrenzend an den zweiten Rahmensteg (9), zwischen diesem und den mindestens zwei Zwischenstegen (10), durch den Schieber (6) hindurch erstreckt.

3. Gurtbandvorrichtung nach Anspruch 1 oder 2, **dadurch gekennzeichnet, dass** zueinander benachbarte Durchdringungen (26) des Gurtbands (7) durch den Schieber (6) jeweils mittels einem der mindestens zwei Zwischenstege (10) voneinander getrennt sind.

4. Gurtbandvorrichtung nach einem der Ansprüche 1 bis 3, **dadurch gekennzeichnet, dass** die mindestens zwei Zwischenstege (10) von dem Gurtband (7) lediglich einseitig umlaufen werden.

5. Gurtbandvorrichtung nach einem der Ansprüche 1 bis 4, **dadurch gekennzeichnet, dass** das Gurtband (7) einstückig ausgebildet ist.

6. Gurtbandvorrichtung nach einem der Ansprüche 1 bis 5, **dadurch gekennzeichnet, dass** das Gurtband (7) elastisch ausgebildet ist und eine Streckdehnung von mindestens 10 % aufweist.

7. Gurtbandvorrichtung nach einem der Ansprüche 1 bis 6, **dadurch gekennzeichnet, dass** das Gurtband (7) zum Befestigen an dem Stellband-Anker (4) eine Ankerschlaufe (18) oder eine Ankerwulst (28) aufweist.

8. Gurtbandvorrichtung nach einem der Ansprüche 1 bis 7, **dadurch gekennzeichnet, dass** das Gurtband (7) einen thermoplastischen Kunststoff aufweist.

9. Gurtbandvorrichtung nach einem der Ansprüche 1 bis 8, **dadurch gekennzeichnet, dass** der erste Rahmensteg (8), der zweite Rahmensteg (9) und die mindestens zwei Zwischenstege (10) in einer Stegebene (12) angeordnet sind.

10. Gurtbandvorrichtung nach einem der Ansprüche 1 bis 9, **dadurch gekennzeichnet, dass** der Schieber (6) eine Positionierhilfe (15) zum automatisierbaren Ausrichten und/oder Festhalten des Schiebers (6) aufweist.

11. Gurtbandvorrichtung nach Anspruch 10, **dadurch gekennzeichnet, dass** die Positionierhilfe (15) an dem ersten Rahmensteg (8) angeordnet ist.

12. Gurtbandvorrichtung nach einem der Ansprüche 1 bis 11, **dadurch gekennzeichnet, dass** der erste Rahmensteg (8) und/oder der zweite Rahmensteg (9) und/oder die mindestens zwei Zwischenstege (10) eine Steg-Rändelung (14) zum Erhöhen einer gegenüber dem Gurtband (7) ausgeübten Reibkraft aufweisen.

13. Gurtbandvorrichtung nach einem der Ansprüche 1 bis 12, **dadurch gekennzeichnet, dass** der Schieber (6) zwei jeweils mit dem ersten Rahmensteg (8), dem zweiten Rahmensteg (9) und den mindestens zwei Zwischenstegen (10) verbundene Rahmenholme (11) aufweist, wobei mindestens einer der zwei Rahmenholme (11) eine Rahmen-Rändelung (13) zum leichteren Verschieben aufweist.

14. Funktionsvorrichtung, aufweisend
- eine Gurtbandvorrichtung (3) gemäß einem der Ansprüche 1 bis 13, und
- ein Funktionselement (2), an dem der Stellband-Anker (4) und der Stellschlaufen-Anker (5) angebracht sind.

15. Funktionsvorrichtung nach Anspruch 14, **dadurch gekennzeichnet, dass** das Funktionselement (2) ein Gesichtsschutzelement und/oder ein Augenschutzelement und/oder ein Atemschutzelement aufweist.

## Claims

1. A webbing strap device of adjustable length, comprising
- an adjustment strap anchor (4),
- an adjustment loop anchor (5) with
-- an adjustment loop eyelet (25),
- a ladder-shaped slider (6) with
-- a first frame crossbar (8),
-- a second frame crossbar (9) spaced from the first frame crossbar (8),
-- at least two intermediate crossbars (10) arranged between the first frame crossbar (8) and the second frame crossbar (9), and
-- at least three strap openings (21, 22, 23) for the passage of webbing strap (7), and
- a webbing strap (7) attached to the first frame crossbar (8) and to the adjustment strap anchor (4),
- wherein the webbing strap (7) extends through the slider (6) between the at least two intermediate crossbars (10) to form an adjustment loop (24), and
- wherein in the region of the adjustment loop (24), the webbing strap (7) extends through the adjustment loop eyelet (25),
**characterized in that**
the webbing strap (7) passes through each of the strap openings (21, 22, 23) at most once and has a slider loop (17) for fixing to the first frame crossbar (8).

2. The webbing strap device as claimed in claim 1, **characterized in that** the webbing strap (7) extends through the slider (6) adjacent to the second frame crossbar (9), between this and the at least two intermediate crossbars (10).

3. The webbing strap device as claimed in claim 1 or 2, **characterized in that** adjacent passages (26) of the webbing strap (7) through the slider (6) are separated from each other by means of one of the at least two intermediate crossbars (10).

4. The webbing strap device as claimed in any of claims 1 to 3, **characterized in that** the webbing strap (7) runs around the at least two intermediate crossbars (10) only on one side.

5. The webbing strap device as claimed in any of claims 1 to 4, **characterized in that** the webbing strap (7) is formed as one piece.

6. The webbing strap device as claimed in any of claims 1 to 5, **characterized in that** the webbing strap (7) is formed so as to be elastic and has a stretch elongation of at least 10%.

7. The webbing strap device as claimed in any of claims 1 to 6, **characterized in that** the webbing strap (7) has an anchor loop (18) or an anchor bead (28) for fixing to the adjustment strap anchor (4).

8. The webbing strap device as claimed in any of claims 1 to 7, **characterized in that** the webbing strap (7) comprises a thermoplastic.

9. The webbing strap device as claimed in any of claims 1 to 8, **characterized in that** the first frame crossbar (8), the second frame crossbar (9) and the at least two intermediate crossbars (10) are arranged in one crossbar plane (12).

10. The webbing strap device as claimed in any of claims 1 to 9, **characterized in that** the slider (6) has a positioning aid (15) for automatable alignment and/or fixing of the slider (6).

11. The webbing strap device as claimed in claim 10, **characterized in that** the positioning aid (15) is arranged on the first frame crossbar (8).

12. The webbing strap device as claimed in any of claims 1 to 11, **characterized in that** the first frame crossbar (8) and/or the second frame crossbar (9) and/or the at least two intermediate crossbars (10) have crossbar knurling (14) to increase the friction against the webbing strap (7).

13. The webbing strap device as claimed in any of claims 1 to 12, **characterized in that** the slider (6) has two frame struts (11) which are each connected to the first frame crossbar (8), the second frame crossbar (9) and the at least two intermediate crossbars (10), wherein at least one of the two frame struts (11) has a frame knurling (13) for easier sliding.

14. A functional device, comprising
- a webbing strap device (3) as claimed in any of claims 1 to 13, and
- a functional element (2) on which the adjustment strap anchor (4) and the adjustment loop anchor (5) are arranged.

15. The functional device as claimed in claim 14, **characterized in that** the functional element (2) comprises a face protection element and/or an eye protection element and/or a respiratory protection element.

## Revendications

1. Dispositif de sangle réglable en longueur, comprenant
- un ancrage de bande de réglage (4),
- un ancrage à boucle de réglage (5) avec
-- un œillet de boucle de réglage (25),
- un coulisseau (6) en forme d'échelle avec
-- une première entretoise de cadre (8),
-- une deuxième entretoise de cadre (9) espacée de la première entretoise de cadre (8),
-- au moins deux entretoises intermédiaires (10) disposées entre la première entretoise de cadre (8) et la deuxième entretoise de cadre (9), et
-- au moins trois ouvertures de sangle (21, 22, 23) pour le passage d'une sangle (7), et
- une sangle (7) fixée à la première entretoise de cadre (8) et à l'ancrage de bande de réglage (4),
- dans lequel la sangle (7) s'étend à travers le coulisseau (6) pour former une boucle de réglage (24) entre les au moins deux entretoises intermédiaires (10), et
- dans laquelle la sangle (7) s'étend à travers l'œillet de boucle de réglage (25) dans la zone de la boucle de réglage (24),
**caractérisé en ce que**
la sangle (7) traverse au maximum une fois chacune des ouvertures de sangle (21, 22, 23) et présente une boucle coulissante (17) pour la fixation sur la première entretoise de cadre (8).

2. Dispositif de sangle selon la revendication 1, **caractérisé en ce que** la sangle (7) s'étend à travers le coulisseau (6) de manière adjacente à la deuxième entretoise de cadre (9), entre celle-ci et les au moins deux entretoises intermédiaires (10).

3. Dispositif de sangle selon la revendication 1 ou 2, **caractérisé en ce que** des pénétrations (26) de la sangle (7) voisines les unes des autres sont séparées les unes des autres par le coulisseau (6) respectivement au moyen d'une des au moins deux entretoises intermédiaires (10).

4. Dispositif de sangle selon l'une quelconque des revendications 1 à 3, **caractérisé en ce que** les au moins deux entretoises intermédiaires (10) sont contournées par la sangle (7) uniquement d'un côté.

5. Dispositif de sangle selon l'une quelconque des revendications 1 à 4, **caractérisé en ce que** la sangle (7) est réalisée en une seule pièce.

6. Dispositif de sangle selon l'une quelconque des revendications 1 à 5, **caractérisé en ce que** la sangle (7) est conçue à être élastique et présente un allongement à la traction d'au moins 10 %.

7. Dispositif de sangle selon l'une quelconque des revendications 1 à 6, **caractérisé en ce que** la sangle (7) présente une boucle d'ancrage (18) ou un bourrelet d'ancrage (28) pour la fixation à l'ancrage de bande de réglage (4).

8. Dispositif de sangle selon l'une quelconque des revendications 1 à 7, **caractérisé en ce que** la sangle (7) présente une matière thermoplastique.

9. Dispositif de sangle selon l'une quelconque des revendications 1 à 8, **caractérisé en ce que** la première entretoise de cadre (8), la deuxième entretoise de cadre (9) et les au moins deux entretoises intermédiaires (10) sont disposées dans un plan d'entretoises (12).

10. Dispositif de sangle selon l'une quelconque des revendications 1 à 9, **caractérisé en ce que** le coulisseau (6) présente une aide au positionnement (15) pour l'orientation et/ou le maintien automatisable du coulisseau (6).

11. Dispositif de sangle selon la revendication 10, **caractérisé en ce que** l'aide au positionnement (15) est disposée sur la première entretoise de cadre (8).

12. Dispositif de sangle selon l'une quelconque des revendications 1 à 11, **caractérisé en ce que** la première entretoise de cadre (8) et/ou la deuxième entretoise de cadre (9) et/ou les au moins deux entretoises intermédiaires (10) présentent un moletage d'entretoise (14) pour augmenter une force de frottement exercée par rapport à la sangle (7).

13. Dispositif de sangle selon l'une quelconque des revendications 1 à 12, **caractérisé en ce que** le coulisseau (6) comporte deux longerons de cadre (11) reliés respectivement à la première entretoise de cadre (8), à la deuxième entretoise de cadre (9) et aux au moins deux entretoises intermédiaires (10), dans lequel au moins un des deux longerons de cadre (11) présente un moletage de cadre (13) pour faciliter le coulissement.

14. Dispositif fonctionnel, comprenant
- un dispositif de sangle (3) selon l'une quelconque des revendications 1 à 13, et
- un élément fonctionnel (2) sur lequel sont montés l'ancrage de bande de réglage (4) et l'ancrage à boucle de réglage (5).

15. Dispositif fonctionnel selon la revendication 14, **caractérisé en ce que** l'élément fonctionnel (2) présente un élément de protection du visage et/ou un élément de protection des yeux et/ou un élément de protection respiratoire.
